# EUROPEAN PATENT SPECIFICATION

(11) **EP 3 774 975 B1**
(45) Date of publication and mention of the grant of the patent: **14.06.2023**
(21) Application number: 19715727.4
(22) Date of filing: 22.03.2019
(51) Int. Cl.: C08G 18/62, A61L 27/18, C08G 18/65, C08G 18/76, C08G 18/32, C08G 18/40, C08G 18/44, A61L 15/26, A61N 1/05

(54) **POLY(ETHER-CARBONATE)-BASED POLYMERS AND MEDICAL DEVICES**
POLYMERE AUF BASIS VON POLY(ETHERCARBONAT) UND MEDIZINISCHE VORRICHTUNGEN
POLYMÈRES À BASE DE POLY(ÉTHER-CARBONATE) ET DISPOSITIFS MÉDICAUX

(30) Priority: 26.03.2018 US 201862648093 P; 20.03.2019 US 201916358939
(43) Date of publication of application: 17.02.2021
(73) Proprietor: Medtronic, Inc., Minneapolis, Minnesota 55432 (US)
(72) Inventor: CHAFFIN, Kimberly A., Minneapolis, Minnesota 55432 (US); CHEN, Xiangji, Minneapolis, Minnesota 55432 (US); JOLLY, Matthew, Minneapolis, Minnesota 55432 (US); LYU, SuPing, Minneapolis, Minnesota 55432 (US); THOR, Peter L., Minneapolis, Minnesota 55432 (US); UNTEREKER, Darrel F., Minneapolis, Minnesota 55432 (US); WANG, Zhaoxu, Minneapolis, Minnesota 55432 (US)
(74) Representative: Zimmermann & Partner Patentanwälte mbB
(86) International application number: PCT/US2019/023540
(87) International publication number: WO 2019/190905

(56) References cited:
- WO-A2-2016/098073
- US-A1- 2001 053 933
- US-A1- 2009 326 077

## Description

### BACKGROUND

There is a need for soft flexible polymers and constructs thereof with improved biostability for implantable medical devices, such as electrical lead insulation, drug infusion catheters, artificial polymer discs, etc. Widely adopted soft 80A durometer polyether-polyurethanes (e.g., those available under the tradenames PELLETHANE, ELASTHANE, TECOTHANE) have desirable hydrolytic stability, mechanical properties, and processing characteristics that have led to their adoption in the implantable medical device industry. These materials are susceptible to oxidation, however, which is generally thought to occur from oxygen-based free radicals, originating from macrophages or foreign body giant cells or catalyzed by metal ions. These radicals can extract a hydrogen atom from the methylene group adjacent to the ether functionality in the polyether soft segment, resulting in chain scission. The oxidative processes observed in vivo with polyurethanes are commonly referred to as Environment Stress Cracking (ESC) and Metal Ion Oxidation (MIO).

Since the observations back in the early 1980's of oxidative degradation of soft polyether-polyurethanes, the implantable medical device industry has continuously explored soft 80A polyurethanes with improved oxidative stability, such as poly(dimethyl siloxane)-polyurethanes or PDMS-polyurethanes (e.g., those available under the trade names ELAST-EON and PURSIL) and polycarbonate-polyurethanes (e.g., that available under the tradename BIONATE). Despite their well-documented improved oxidative stability, however, these materials have not been widely adopted for various reasons.

Polycarbonate-polyurethanes show better oxidation stability compared to polyether-polyurethanes; however, they are still susceptible to oxidation to a certain degree. Also, the carbonate groups in the soft segment is generally considered hydrolysable under certain conditions. PDMS-polyurethanes have recently been proven to be susceptible to hydrolysis resulting in decreasing mechanical strength. It should be noted that hydrolysis, unlike oxidation, typically occurs throughout the polymer as water can readily diffuse into the bulk. Consequently, bulk mechanical properties are affected by hydrolysis, whereas cell-mediated oxidation takes place primarily at the surface.

Polymers are disclosed in WO 2016/098073, US 2009/326007 and US 2001/053933.

Thus, other approaches of making better polymers for use in medical devices are needed.

### SUMMARY

The present disclosure provides a poly(ether-carbonate)-based polymer and a medical device that includes such polymer.

In one embodiment, a poly(ether-carbonate)-based polymer is provided that includes urethane, urea, or urethane-urea groups, hard segments, and soft segments, wherein the soft segments include poly(ether-carbonate) residues and at least one of polyisobutylene (PIB) residues and hydrogenated polybutadiene residues, and the hard segments include diisocyanate residues and optionally chain extender residues.

The use of poly(ether-carbonate) with PIB or hydrogenated polybutadiene as soft segments results in a polymer having surprisingly better mechanical properties, particularly tear strength, and chemical stability compared to the similar polymers without the poly(ether-carbonate). Tear strength is very important for certain medical device applications. For example, a lead insulation tubing with low tear strength will tend to split.

In another embodiment, a medical device is provided that includes a polymeric region including the poly(ether-carbonate)-based polymer described herein. Such medical devices include, for example, an implantable electrical lead, an implantable electrical pulse generator, or an implantable mechanical device.

Polymers of the present disclosure are elastomers. An "elastomer" is a polymer that is capable of being stretched to approximately twice its original length and retracting to approximately its original length upon release. Polymers of the present disclosure can be made of two or more different monomers. They can be random, alternating, block, star-shaped block, segmented copolymers, or combinations thereof. Preferably, the polymers are segmented copolymers (i.e., containing a multiplicity of both hard and soft domains or segments on any polymer chain) and are comprised substantially of alternating relatively soft segments and relatively hard segments.

As used herein, a "hard" segment is one that is crystalline at use temperature, or amorphous with a glass transition temperature above use temperature, or when in the water saturated state at body temperature, a hard segment has a Tg of about 30°C (below body temperature, but above that of the soft segments -100°C to -30°C), and a "soft" segment is one that is amorphous with a glass transition temperature below use temperature (i.e., rubbery). A crystalline or glassy moiety or hard segment is one that adds considerable strength and higher modulus to the polymer. Similarly, a rubbery moiety or soft segment is one that adds flexibility and lower modulus, but may add strength particularly if it undergoes strain crystallization, for example. The random or alternating soft and hard segments are linked by urethane groups, urea groups, or urethane-urea groups, and the polymers may be terminated by hydroxyl, amine, and/or isocyanate groups or surface modified end groups.

As used herein, "alkyl" refers to a monovalent group that is a radical of an alkane and includes straight-chain, branched, cyclic, and bicyclic alkyl groups, and combinations thereof, including both unsubstituted and substituted alkyl groups. Unless otherwise indicated, the alkyl groups typically contain from 1 to 16 carbon atoms. In some embodiments, the alkyl groups contain 1 to 10 carbon atoms, 1 to 6 carbon atoms, 1 to 4 carbon atoms, or 1 to 3 carbon atoms. In some embodiments, the cycloalkyl groups contain 3 to 16 carbon atoms, 3 to 10 carbon atoms, or 3 to 6 carbon atoms. Examples of "alkyl" groups include, but are not limited to, methyl, ethyl, n-propyl, n-butyl, n-pentyl, isobutyl, t-butyl, isopropyl, n-octyl, n-heptyl, ethylhexyl, cyclopentyl, cyclohexyl, cycloheptyl, adamantyl, norbornyl.

As used herein, "alkenyl" refers to a monovalent group that is a radical of an alkene and includes straight-chain, branched, cyclic, and bicyclic alkenyl groups, and combinations thereof, including both unsubstituted and substituted alkenyl groups. Unless otherwise indicated, the alkenyl groups typically contain from 2 to 16 carbon atoms. In some embodiments, the alkenyl groups contain 2 to 10 carbon atoms, 2 to 6 carbon atoms, or 2 to 4 carbon atoms. In some embodiments, the cycloalkenyl groups contain 3 to 16 carbon atoms, 3 to 10 carbon atoms, or 3 to 6 carbon atoms. Examples of "alkenyl" groups include, but are not limited to, ethenyl, n-propenyl, n-butenyl, n-pentenyl, isobutenyl, t-butenyl, cyclohexenyl.

As used herein, "alkynyl" refers to a monovalent group that is a radical of an alkyne and includes straight-chain, branched, cyclic, and bicyclic alkynyl groups, and combinations thereof, including both unsubstituted and substituted alkynyl groups. Unless otherwise indicated, the alkynyl groups typically contain from 2 to 16 carbon atoms. In some embodiments, the alkynyl groups contain 2 to 10 carbon atoms, 2 to 6 carbon atoms, or 2 to 4 carbon atoms. In some embodiments, the alkynyl groups contain 3 to 16 carbon atoms, 3 to 10 carbon atoms, or 3 to 6 carbon atoms. Examples of "alkynyl" groups include, but are not limited to, ethynyl, n-propynyl, n-butynyl, n-pentynyl, isobutynyl, t-butynyl, cyclohexynyl.

The term "aryl" refers to a monovalent group that is aromatic and, optionally, carbocyclic. The aryl has at least one aromatic ring. Any additional rings can be unsaturated, partially saturated, saturated, or aromatic. Optionally, the aromatic ring can have one or more additional carbocyclic rings that are fused to the aromatic ring. Unless otherwise indicated, the aryl groups typically contain from 6 to 18 carbon atoms. In some embodiments, the aryl groups contain 6 to 16 carbon atoms, 6 to 12 carbon atoms, or 6 to 10 carbon atoms. Examples of an aryl group include phenyl, naphthyl, biphenyl, phenanthryl, and anthracyl.

An "alkylene" (including a "cycloalkylene"), an "alkenylene" (including a "cycloalkenylene"), an "alkynylene" (including a "cycloalkynylene") refer to divalent groups that are radicals of an alkane, alkene, and alkyne, respectively, and includes groups that are linear, branched, cyclic, bicyclic, or a combination thereof. The numbers of carbon atoms are as described above for alkyl, alkenyl, and alkynyl.

The term "arylene" refers to a divalent group that is aromatic and, optionally, carbocyclic. The arylene has at least one aromatic ring. Optionally, the aromatic ring can have one or more additional carbocyclic rings that are fused to the aromatic ring. Any additional rings can be unsaturated, partially saturated, or saturated. Unless otherwise specified, arylene groups often have 6 to 20 carbon atoms, 6 to 18 carbon atoms, 6 to 16 carbon atoms, 6 to 12 carbon atoms, or 6 to 10 carbon atoms.

A "medical device" may be defined as a device that has surfaces that contact blood or other bodily fluids in the course of their operation. This can include, for example, extracorporeal devices for use in surgery, such as blood oxygenators, blood pumps, blood sensors, tubing used to carry blood, which contact blood which is then returned to the patient. This can also include endoprostheses implanted in blood contact in a human or animal body such as vascular grafts, stents, stent grafts, medical electrical leads, indwelling catheters, heart valves, that are implanted in blood vessels or in the heart. This can also include devices for temporary intravascular use such as catheters, guide wires, balloons, which are inserted into the blood vessels or the heart for purposes of monitoring or repair. Thus, a medical device may be an implantable device or an insertable device.

Herein, the term "comprises" and variations thereof do not have a limiting meaning where these terms appear in the description and claims. Such terms will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether they materially affect the activity or action of the listed elements. Any of the elements or combinations of elements that are recited in this specification in open-ended language (e.g., comprise and derivatives thereof), are considered to additionally be recited in closed-ended language (e.g., consist and derivatives thereof) and in partially closed-ended language (e.g., consist essentially, and derivatives thereof).

The words "preferred" and "preferably" refer to embodiments of the disclosure that may afford certain benefits, under certain circumstances. However, other claims may also be preferred, under the same or other circumstances. Furthermore, the recitation of one or more preferred claims does not imply that other claims are not useful, and is not intended to exclude other claims from the scope of the disclosure.

In this application, terms such as "a," "an," and "the" are not intended to refer to only a singular entity, but include the general class of which a specific example may be used for illustration. The terms "a," "an," and "the" are used interchangeably with the term "at least one." The phrases "at least one of" and "comprises at least one of" followed by a list refers to any one of the items in the list and any combination of two or more items in the list.

As used herein, the term "or" is generally employed in its usual sense including "and/or" unless the content clearly dictates otherwise.

The term "and/or" means one or all of the listed elements or a combination of any two or more of the listed elements.

Also herein, all numbers are assumed to be modified by the term "about" and in certain embodiments, preferably, by the term "exactly." As used herein in connection with a measured quantity, the term "about" refers to that variation in the measured quantity as would be expected by the skilled artisan making the measurement and exercising a level of care commensurate with the objective of the measurement and the precision of the measuring equipment used. Herein, "up to" a number (e.g., up to 50) includes the number (e.g., 50).

Also herein, the recitations of numerical ranges by endpoints include all numbers subsumed within that range as well as the endpoints (e.g., 1 to 5 includes 1, 1.5, 2, 2.75, 3, 3.80, 4, 5, etc.).

As used herein, the terms "ambient temperature" or "room temperature" refer to a temperature of 20°C to 25°C or 22°C to 25°C.

The term "in the range" or "within a range" (and similar statements) includes the endpoints of the stated range.

Groupings of alternative elements or embodiments disclosed herein are not to be construed as limitations. Each group member may be referred to and claimed individually or in any combination with other members of the group or other elements found therein. It is anticipated that one or more members of a group may be included in, or deleted from, a group for reasons of convenience and/or patentability. When any such inclusion or deletion occurs, the specification is herein deemed to contain the group as modified thus fulfilling the written description of all Markush groups used in the appended claims.

When a group is present more than once in a formula described herein, each group is "independently" selected, whether specifically stated or not. For example, when more than one R group is present in a formula, each R group is independently selected.

Reference throughout this specification to "one embodiment," "an embodiment," "certain embodiments," or "some embodiments," etc., means that a particular feature, configuration, composition, or characteristic described in connection with the embodiment is included in at least one embodiment of the invention. Thus, the appearances of such phrases in various places throughout this specification are not necessarily referring to the same embodiment of the invention. Furthermore, the particular features, configurations, compositions, or characteristics may be combined in any suitable manner in one or more embodiments.

The above summary of the present disclosure is not intended to describe each disclosed embodiment or every implementation of the present invention. The description that follows more particularly exemplifies illustrative embodiments. In several places throughout the application, guidance is provided through lists of examples, which examples may be used in various combinations. In each instance, the recited list serves only as a representative group and should not be interpreted as an exclusive list. Thus, the scope of the present disclosure should not be limited to the specific illustrative structures described herein, but rather extends at least to the structures described by the language of the claims, and the equivalents of those structures. Any of the elements that are positively recited in this specification as alternatives may be explicitly included in the claims or excluded from the claims, in any combination as desired. Although various theories and possible mechanisms may have been discussed herein, in no event should such discussions serve to limit the claimable subject matter.

### BRIEF DESCRIPTION OF THE FIGURES

FIG. 1 is a diagram of a lead which incorporates a polymer of the present disclosure.
FIG. 2 is a cross-sectional view of the lead body of the lead shown in FIG. 1.
FIG. 3 is a cross-section of an exemplary lead body.
FIG. 4 is a diagram of a neurological electrical lead which incorporates a polymer of the present disclosure.
FIG. 5 is a diagram of a neurological drug delivery lead which incorporates a polymer of the present disclosure.
FIG. 6 is an SEM image of PIB-PEC-PU at 400x magnification after 8 weeks of oxidation aging.
FIG. 7 is an SEM image of PIB-PTMO-PU (comparative polymer) at 400x magnification after 8 weeks of oxidation aging.
FIG. 8 is an SEM image of PIB-PU (comparative polymer) at 400x magnification after 8 weeks of oxidation aging.

### DETAILED DESCRIPTION OF ILLUSTRATIVE EMBODIMENTS

This disclosure provides a poly(ether-carbonate)-based polymer and a medical device that includes such polymer.

In one embodiment, a poly(ether-carbonate)-based polymer is provided that includes urethane, urea, or urethane-urea groups, hard segments, and soft segments, wherein the soft segments include poly(ether-carbonate) residues and at least one of polyisobutylene (PIB) residues and hydrogenated polybutadiene residues, and the hard segments include diisocyanate residues and optionally chain extender residues. In certain embodiments, a poly(ether-carbonate)-based ("PEC-based") polymer of the present disclosure includes urethane groups, and in certain embodiments, no urea groups. Such polymers are elastomers.

The use of poly(ether-carbonate) with PIB or hydrogenated polybutadiene as soft segments results in a polymer having surprisingly better mechanical properties, particularly tear strength, compared to the similar polymers without the poly(ether-carbonate). Tear strength is very important for certain medical device applications. For example, a lead insulation tubing with low tear strength will tend to split.

In certain embodiments, a poly(ether-carbonate)-based polymer of the present disclosure has a greater tear strength than a comparative polymer without poly(ether-carbonate) residues, wherein the comparative polymer has a shore durometer within 50% to 150% of the shore durometer of the poly(ether-carbonate)-based polymer. In certain embodiments, a poly(ether-carbonate)-based polymer of the present disclosure has a tear strength at least 10%, or at least 20%, greater than a comparative polymer without poly(ether-carbonate) residues, wherein the comparative polymer has a shore durometer within 50% to 150% of the shore durometer of the poly(ether-carbonate)-based polymer.

The use of poly(ether-carbonate) with PIB or hydrogenated polybutadiene as soft segments results in a polymer having surprisingly better chemical stability compared to similar polymers without the poly(ether-carbonate).

In certain embodiments, a poly(ether-carbonate)-based polymer of the present disclosure has greater overall chemical stability than a comparative polymer with poly(tetramethylene oxide) residues instead of poly(ether-carbonate) residues, wherein the comparative polymer has an amount of poly(tetramethylene oxide) residues equal to an amount of poly(ether-carbonate) residues in the poly(ether-carbonate)-based polymer, and wherein the comparative polymer has a shore durometer within 50% to 150% of the shore durometer of the poly(ether-carbonate)-based polymer.

In certain embodiments, a poly(ether-carbonate)-based polymer of the present disclosure has a weight average molecular weight of at least 10,000 Daltons, as determined by gel permeation chromatography with multiangle laser light scattering detection. In certain embodiments, a poly(ether-carbonate)-based polymer of the present disclosure has a weight average molecular weight of up to 2,000,000 Daltons, or up to 1,000,000 Daltons, as determined by gel permeation chromatography with multiangle laser light scattering detection. In certain embodiments, a poly(ether-carbonate)-based polymer of the present disclosure has a weight average molecular weight of 10,000 Daltons to 2,000,000 Daltons, or 10,000 Daltons to 1,000,000 Daltons, as determined by gel permeation chromatography with multiangle laser light scattering detection.

A polyurethane includes linkages of the following structure: 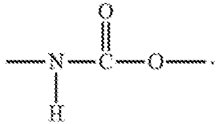 Such polymers can be made using conventional techniques. Typically, polyurethanes are made by a process in which one or more polyfunctional isocyanates (e.g., diisocyanates, including both aliphatic and aromatic diisocyanates) is reacted with one or more polyols (e.g., diols) to form a prepolymer. The resulting prepolymer can be further reacted with a chain extender, such as a diol.

A polyurea includes linkages of the following structure: 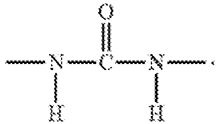 Such polymers can be made using conventional techniques. Typically, polyureas are made by a process in which one or more polyfunctional isocyanates (e.g., diisocyanates, including both aliphatic and aromatic diisocyanates) is reacted with one or more polyamines (e.g., diamines) to form a prepolymer. The resulting prepolymer can be further reacted with a chain extender, such as a diol.

A polyurethane-urea includes both of the above-described linkages. Such polymers can be made using conventional techniques. Typically, polyurethane-ureas are made by a process in which one or more polyfunctional isocyanates (e.g., diisocyanates, including both aliphatic and aromatic diisocyanates) is reacted with one or more polyamines (e.g., diamines) and one or more polyols (e.g., diols) to form a prepolymer. The resulting prepolymer can be further reacted with a chain extender, such as a diol.

### Soft Segments

Soft segments of the polymers of the present disclosure include poly(ether-carbonate) residues and at least one of polyisobutylene (PIB) residues and hydrogenated polybutadiene residues (i.e., polyisobutylene residues and/or hydrogenated polybutadiene residues). In certain embodiments, soft segments of the polymers of the present disclosure consist essentially of poly(ether-carbonate) residues and polyisobutylene and/or hydrogenated polybutadiene residues, wherein there may be small amounts of other soft segment residues but only minor amounts that do not change the properties of the polymer. In certain embodiments, soft segments of the polymers of the present disclosure consist of poly(ether-carbonate) residues and polyisobutylene and/or hydrogenated polybutadiene residues, wherein there no other soft segment residues. In certain embodiments, soft segments of the polymers of the present disclosure consist of poly(ether-carbonate) residues and polyisobutylene residues.

In certain embodiments, polyisobutylene and/or hydrogenated polybutadiene soft segment residues can be derived from a diol, a diamine, or a combination thereof. In certain embodiments, the polyisobutylene and/or hydrogenated polybutadiene residues are derived from one or more diols.

In certain embodiments, the polyisobutylene residues include initiator residues. The term "initiator residue" refers to a difunctional chemical moiety, that links two linear chains of a polymer. For example, the polyisobutylene residues can be derived from a polyisobutylene of the following formula: 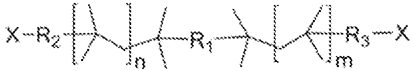 wherein Ri is an initiator residue; each X is independently -OH, -NH₂, or -NHR₄; n = 0-500; m = 0-500; each of R₂ and R₃ is selected from a (C1-C16)alkylene, a (C3-C16)cycloalkylene, a (C2-C16)alkenylene, a (C3-C16)cycloalkenylene, a (C2-C16)alkynylene, a (C3-C16)cycloalkynylene, or a (C6-C18)arylene, wherein for each occurrence of R₂ or R₃ is independently optionally substituted with one or more groups selected from halo, cyano, nitro, dialkylamino, trialkylamino, (C1-Cl6)alkoxy, and (C1-C16 haloalkyl); and R₄ is selected from a (C1-C16)alkyl, a (C3-C16)cycloalkyl, a (C2-C16)alkenyl, a (C3-C16)cycloalkenyl, a (C2-C16)alkynyl, a (C3-C16)cycloalkynyl, or a (C6-C18)aryl.

Such compounds are referred to herein as "PIB" or polyisobutylene, i.e., a compound formed by polymerization of isobutylene and includes an initiator residue.

In certain embodiments, the polyisobutylene residues can be derived from a polyisobutylene diol of the following formula: 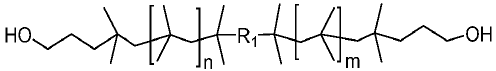 wherein R₁ is an initiator residue, n = 0-500, and m = 0-500.

Such initiator residues may include aromatic residues, aliphatic residues, or combinations thereof. Examples of initiators include 5-tert-butyl-1,3-bis(1-methoxy-1-methylethyl)-benzene (or "hindered dicumyl ether" or "HDCE"), 1,3-bis(1-chloro-1-methylethyl)-5-(1-dimethylethyl)benzene (or, alternatively, known as 1,3-bis(1-chloro-1-methylethyl)-5-tert-butylbenzene or "hindered dicumyl chloride" or "HDCC"), 2,6-dichloro-2,4,4,6-tetramethylheptane, and 2,5-dichloro-2,5-dimethylhexane.

Such initiator residues may include aromatic residues. In certain embodiments, the polyisobutylene residues include aromatic-containing polyisobutylene residues. For example, the polyisobutylene residues can be derived from a polyisobutylene diol of the following formula: 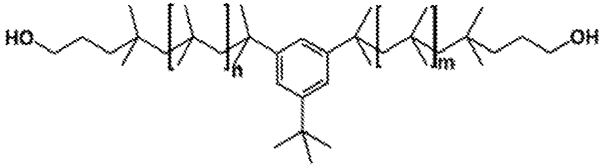 wherein n = 0-500 and m = 0-500.

In certain embodiments, poly(ether-carbonate) soft segment residues are derived from a poly(ether-carbonate) diol, a poly(ether-carbonate) diamine, or a combination thereof. In certain embodiments, poly(ether-carbonate) soft segment residues are derived from one or more poly(ether-carbonate) diols.

In certain embodiments, the poly(ether-carbonate) residues are derived from a poly(ether-carbonate) compound of the following formula: 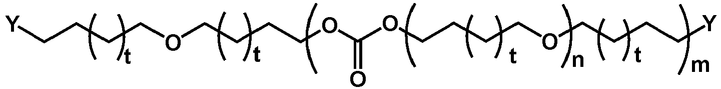 wherein each Y is independently -OH, -NH₂, or -NHR₅, t = 1-9, n = 0-40, and m = 1-40. Each of R₅ is selected from a (C1-C16)alkyl, a (C3-C16)cycloalkyl, a (C2-C16)alkenyl, a (C3-C16)cycloalkenyl, a (C2-C16)alkynyl, a (C3-C16)cycloalkynyl, or a (C6-C18)aryl.

In certain embodiments, the poly(ether-carbonate) residues are derived from a poly(ether-carbonate) diol of the following formula: 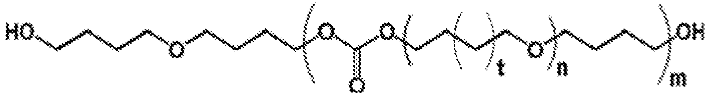 wherein t = 1-9, n = 0-40, and m = 1-40.

Thus, the soft segments of the polymers of the present disclosure include copolymers of ether and carbonate groups. This is distinct from polymers that include pure polyether soft segments pure polycarbonate soft segments separated by hard segments. While polymers of the present disclosure may include a small amount of pure polyether soft segments and pure polycarbonate soft segments, the majority (typically, greater than 50 wt-%) of the soft segments include co-poly ether-poly carbonate soft segments.

In certain embodiments, the weight ratio of polyisobutylene and/or hydrogenated polybutadiene residues to poly(ether-carbonate) residues is up to 99:1, up to 95:5, or up to 90:10. In certain embodiments, the weight ratio of polyisobutylene and/or hydrogenated polybutadiene residues to poly(ether-carbonate) residues is at least 1:99, at least 50:50, or at least 80:20. In certain embodiments, the weight ratio of polyisobutylene and/or hydrogenated polybutadiene residues to poly(ether-carbonate) residues is within a range of 99: 1 to 1:99, within a range of 95:5 to 50:50, or within a range of 90:10 to 80:20.

In certain embodiments, soft segments may also include additional residues selected from polybutadiene residues, hydrogenated polybutadiene residues, polycarbonate residues, polyether residues, polyester residues, polysiloxane residues, and combinations thereof, which may be provided by diols, diamines, or combinations thereof. If present, such additional soft segment residues are present in an amount of no more than 60% of final polymer by weight.

### Hard Segments

A poly(ether-carbonate)-based polymer of the present disclosure includes hard segments including diisocyanate residues and optionally chain extender residues.

In certain embodiments, the diisocyanate residues are derived from 4,4'-methylenediphenyl diisocyanate, 4,4'-methylenebis(cyclohexyl isocyanate), toluene diisocyanate, 1,5-naphthalene diisocyanate, para-phenylene diisocyanate, 3,3'-tolidene-4,4'-diisocyanate, 3,3'-dimethyldiphenylmethane-4,4'-diisocyanate, and combinations thereof. In certain embodiments, the diisocyanate residues are derived from 4,4'-methylenediphenyl diisocyanate (MDI) to contribute to a polymer having excellent hydrolysis stability.

In certain embodiments, hard segments include chain extender residues. In certain embodiments, the chain extender residues are derived from at least one chain extender selected from an aliphatic diol, an aromatic diol, an aliphatic diamine, an aromatic diamine, and a combination thereof. In certain embodiments, the chain extender includes one or more aliphatic diols. In certain embodiments, the aliphatic diol chain extender includes one or more alpha,omega-(C1-C16)alkane diols. In certain embodiments, the chain extender includes 1,2-ethane diol, 1,4-butanediol, 1,6-hexanediol, or combinations thereof.

In certain embodiments, the weight ratio of soft segments to hard segments in a poly(ether-carbonate)-based polymer of the present disclosure is up to 90: 10, up to 80:20, or up to 70:30. In certain embodiments, the weight ratio of soft segments to hard segments in a poly(ether-carbonate)-based polymer of the present disclosure is at least 10:90, at least 30:70, or at least 40:60. In certain embodiments, the weight ratio of soft segments to hard segments in a poly(ether-carbonate)-based polymer of the present disclosure is within a range of 90: 10 to 10:90, within a range of 80:20 to 30:70, or within a range of 70:30 to 40:60.

### Medical Devices

The present disclosure also provides medical devices. Such devices include a polymeric region including the poly(ether-carbonate)-based polymer described herein. Such medical devices include, for example, an implantable electrical lead, an implantable electrical pulse generator, or an implantable mechanical device (e.g., ventricle assistance device, pump, and an artificial valve such as a prosthetic heart valve).

A prosthetic heart valve replaces the function of a native heart valve such that the prosthetic valve regulates the flow of blood through the heart. In one aspect, a prosthetic heart valve includes a base and a plurality of polymeric leaflets. Each leaflet has a root portion coupled to the base, and each leaflet has an edge portion substantially opposite the root portion and movable relative to the root portion to coat with a respective edge portion of at least one of the other leaflets of the plurality of leaflets.

Particularly suitable medical devices include medical leads. Medical leads are used to transmit electrical signals to and from medical devices such as pacemakers and neurostimulators, for example. The lead body is usually made from a piece of polymeric tubing having a round cross-section exterior and a round cross-section lumen. Typically, a coiled metallic electrical conductor having a round cross-section is placed in the lumen completely filling it. The tubing protects and insulates the conductor. The coiled conductor can usually receive a stylet to help position and place the lead during implantation. There are many examples of medical electrical leads, including, for example, those described in U.S. Pat. Nos. 6,785,576 (Vemess), 5,303,704 (Molacek et al.), 5,999,858 (Sommer et al.), 6,149,678 (DiDomenico et al.), 4,947,866 (Lessar et al.), 5,922,014 (Warman et al.), 5,628,778 (Kruse et al.), 4,497,326 (Curry), 5,443,492 (Stokes et al.), and 7,860,580 (Falk et al.).

In certain embodiments, the medical device is an implantable electrical lead. In certain embodiments, the electrical lead is a cardiac lead or a neurostimulation lead. In certain embodiments, the implantable electrical lead includes an electrical conductor and a layer including a poly(ether-carbonate)-based polymer of the present disclosure disposed on the electrical conductor. In certain embodiments, the polymeric region further includes a therapeutic agent (i.e., a drug).

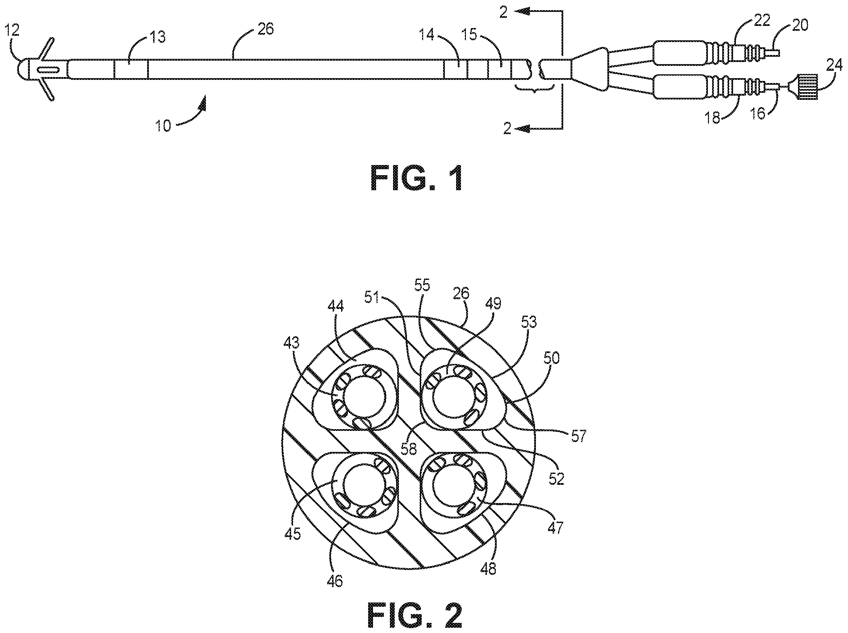

As an exemplary embodiment of a medical electrical lead, FIG. 1 is a diagram of a lead assembly 10, which incorporates a polymer of the present disclosure. The lead body 26 carries four electrodes including ventricular electrodes 12 and 13 and atrial electrodes 14 and 15. Within the lead body are four conductors, one coupled to each of the electrodes and extending proximally to a corresponding electrical connector. The proximal end of the lead assembly 10 has a dual in-line connector assembly including connector pin 16, coupled to electrode 12, connector ring 18, coupled to electrode 13, connector pin 20, coupled to electrode 14 and connector ring 22, coupled to electrode 15. A stylet 24 may be 25 inserted into the lead through pin 16 to stiffen it as an aid to implantation.

Lead body 26 in FIG. 1 is preferably fabricated of silicone rubber, polyurethane or other implantable polymer. In particular, lead body 26 is preferably fabricated of a polymer of the present disclosure.

Electrodes 12, 13, 14, and 15 in FIG. 1 are preferably fabricated of platinum alloy or 30 other biocompatible metal. Connectors 16, 18, 20, and 22 are preferably fabricated of stainless steel or other biocompatible metal.

As illustrated, the lead includes electrodes which may serve as means for delivery of stimulation pulses and as means for sensing physiological electrical signals. It should also be understood that a lead according to the present disclosure may also include means for sensing other physiological parameters, such as pressure, oxygen saturation, temperature, or pH. The lead may include electrodes only, other physiologic sensors only, or a combination of both.

FIG. 2 is a cross-section through the lead body 26. In this view, it can be seen that lead body 26 is provided with four pie-shaped or generally triangular lumens. The first lumen 44 contains a first coiled conductor 43. The second lumen 46 contains a second coiled conductor 45. The third lumen 48 contains a third coiled conductor 47. The fourth lumen 50 contains a fourth coiled conductor 49. The conductors 43, 45, 47, and 49 are preferably fabricated of MP35N alloy or other biocompatible metal. In the drawing each coiled conductor is shown as a multi-filar coil; however, monofilar coils are useful as well.

One of the four conductors is coupled to pin 16 and also serves to receive a stylet. The lead body may employ the multi-lumen configuration illustrated over its entire length, with two of the lumens unused distal to electrodes 14 and 15. Alternatively, a transition to a lead body having a coaxial or side by side two-lumen configuration as typically used in bipolar pacing leads may occur at or distal to electrodes 14 and 15.
As seen in cross section, the representative fourth lumen 50 has three walls each having a radius of curvature substantially greater than the radius of curvature of the conductor coil. These walls include two substantially planar walls 51 and 52 each extending along a radius of the body and an outer curved wall 53, extending along the outer circumference of the lead body. The walls are joined to one another along comers 55, 57, and 58 each of which have a radius of curvature substantially less than the radius of curvature of the conductor coils, as seen in this cross-section.

In certain embodiments, contact between a coil of a conductor and the inner surface of a lumen will be limited to those portions of the inner surfaces of the lumen which have a substantially greater radius of curvature than the conductor coil. Contact will thus be limited to discrete points of contact, rather than along substantial lengths of the individual coils, as would occur in prior leads employing circular coils and circular lumens of similar sizes. Contact will occur only along walls 51, 52 and 53, and not in comers 55, 57 and 59. Along the length of the lead, individual coils will contact various points on all three walls 51, 52, and 53.

The present medical electrical lead includes a polymeric material of the present disclosure as part or all of lead body 26, but theoretically this could apply to any insulator on the lead body.

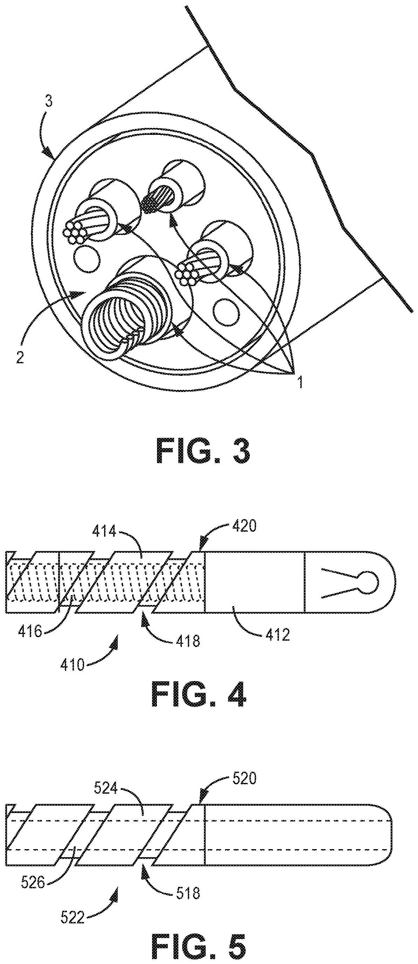

FIG. 3 is a cross-section of another exemplary lead body showing several insulation layers: a primary insulation layer 1, which encapsulates the conductors; a secondary insulation layer 2, which contains the lumens for the conductors; and a tertiary outer insulation layer 3. The polymer of this disclosure forms part or all of any of these insulation layers.

Medical, neurological leads are used for insertion into the human body, for transmission of therapeutic agents (i.e., drugs) and/or electrical signals to body organs such as the spinal cord or brain, for acute and chronic pain relief, acute and chronic treatment of disease. The leads are used in programmable, electronic, implantable devices which deliver drugs and/or electrical stimulation in programs of therapy for the benefit of mankind.

Implantable electrical devices are capable of relieving chronic, inoperable pain by interfering with the transmission of pain signals in the spinal cord and brain. Implantable drug delivery devices are capable of delivering pain relieving drugs to the same dramatic effect. Both types of devices are also capable of new therapies for treatment of a variety of diseases. An advantage of the electrical devices is that typically no drugs are necessary. With the drug delivery devices, an advantage is that drug dosages are reduced relative to other therapies because the drugs are delivered directly to desired locations of therapy, rather than in remote locations such as the blood vessels of the extremities, and without concern for bodily elimination or chemical interaction.

With the electrical devices, electrical stimulation is typically delivered from the devices to the body through wired leads, to electrodes. The electrodes are located on and exposed to the body on the distal extremity of the leads, and the leads typically extend into and along the epidural space of the spinal cord, or into the brain at surgically drilled boreholes. The leads may also be subcutaneous where necessary. As an example, leads may extend from devices implanted above the clavicles, under the skin, to a bore hole atop the skull, and thence deep into brain tissue.

With the drug delivery devices, catheters, which for purposes of this description are also considered "neurological leads," extend in similar ways. Leads in the described applications are typically smooth walled, plastic, tubular members, although variation is possible. There are many examples of medical neurological leads, including, for example, those described in U.S. Pat. Nos. 5,058,584 (Bourgeois), 5,865,843 (Baudino), U.S. Pat. Pub. No. 2008/0275429 (Sage).

Medical neurological leads include, for example, paddle leads, in-line cylindrical leads, and drug delivery catheters. These leads/catheters can be placed in numerous locations. Electrode leads are used in the epidural space, within the brain itself, in the sacral root, and within blood vessels. Cuff-type electrodes, as in U.S. Pat. No. 5,282,468 (Klepinski), can be mounted around nerve bundles or fibers. Drug delivery catheters can be placed in/adjacent the spinal column or any location within the vascular system. The polymeric material of the present disclosure may be used as all or part of the lead body, as insulation, as an inner or outer layer.

Referring to FIGS. 4 and 5, an exemplary lead of the disclosure includes a distal portion 10, and associated central and proximal portions not shown. As known to persons of ordinary skill in the art, if electrical, the lead may connect to an electrical signal generating device (hereafter "a signal generator"), which may or may not be implantable in whole or in part into the human body. If the lead is a drug delivery lead, the lead may connect to a drug pump, which also may or may not be implantable. In either case, the lead is intended to have at least a portion engaged in the tissue of the body. Depending on the application, the lead may engage tissue in the proximal, central, or distal portions of the lead. The lead may or may not enter the epidural space which surrounds the spinal cord, or the lead may enter the brain through the skull. Generally, the lead is substantially elongated, with the dimension of its length one hundred or more times the dimension of its width.

Again, if electrical, as in FIG. 4, the lead 410 may include one or more electrodes, such as an electrode designated 412. The electrode may be annular, surrounding the lead 30 body, or in other shape or form. If a drug delivery lead, as in FIG. 5, the lead 522 may include one or more openings for transmission of drugs from the drug pump to the body, in the place of electrodes, or in addition to electrodes.

The lead 410 or 522 is desirably, generally circular in cross-section, although variations are within contemplation. Focusing on an electrical lead of FIG. 4, for illustration, an insulating, annular, external lead sheath or body 414 surrounds an electrically transmissive internal core 416, shown in phantom. The core 416 frequently takes the form of a helically wound or coiled wire, interconnected to the distal electrode(s) and the proximal signal generator. The wire has a direction of its winding, which is right hand or left hand, clockwise or counterclockwise. As desired, although not presently contemplated, the lead may also include additional intermediate or other layers, or other components.

FIG 4. shows an electrical lead having a helical groove 418, and associated helical land 420. FIG. 5 shows a drug delivery lead or catheter having a liquid insulating, annular, external lead sheath or body 524 surrounds a liquid transmissive internal and open core or passage 526, shown in phantom.

### EXAMPLES

Objects and advantages of the disclosure are further illustrated by the examples provided herein. The particular materials and amounts thereof recited in these examples, as well as other conditions and details, are merely illustrative and are not intended to be limiting. The person of ordinary skill in the art, after carefully reviewing the entirety of this disclosure, will be able to use materials and conditions in addition to those specifically described in the examples.

### Materials

4,4'-Methylenebis(phenyl isocyanate) (MDI, Aldrich, 98%), 1,4-butanediol (BDO, Aldrich, 99%), polycarbonate diol (PC-diol, ETERNACOLL UH200, 2000 g·mol⁻¹), poly(ether-carbonate) diol (PEC-diol, ETERNACOLL UT200, 2000 g·mol⁻¹), poly(tetramethylene oxide) (PTMO) diol (Aldrich, 1000 g·mol⁻¹), Hydrogenated polybutadiene (HPB) diol (Cray Valley, 3000 g·mol⁻¹), Stannous octoate (Aldrich, 92.5%), 1-Methyl-2-pyrrolidinone(NMP, anhydrous, Aldrich, 99.5%), toluene (anhydrous, Aldrich, 99.8%), N,N-dimethylacetamide (DMAc, anhydrous, Aldrich, 99.8%), tetrahydrofuran (THF, 99.9%), and methanol (Fisher, 99.8%) were used as received.

Polyisobutylene (PIB) diol (3000 g·mol⁻¹) was made from the initiator, 1,3-di(2-methoxypropyl)-5-tert-butylbenzene (Molbase, 99 %) in a reported two-step procedure (Ivan et al. J Polym Sci A Polym Chem 1990, 28, 89-104).

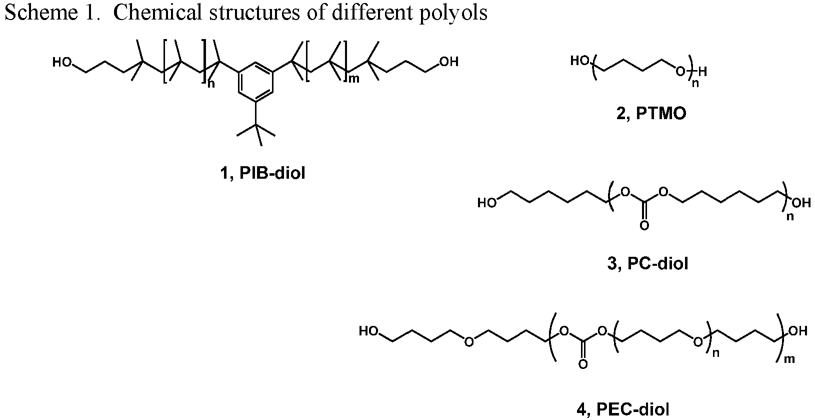

### Characterization Methods

Nuclear Magnetic Resonance (NMR) experiments were conducted on Bruker Avance III HD spectrometer (400 MHz). Samples were dissolved in either CDCl₃ or THF*d₈* for ¹H or ¹³C NMR.

Molecular weights of polymers were determined via a size exclusion chromatography (SEC) system composed of an Agilent 1260 injection system, a Wyatt OPTILAB T-Rex refractive detector and a Wyatt DAWN Heleos II multiangle laser scattering detector. The data were processed by Astra 7. THF was the eluent, which was operated at 40 °C.

Tensile testing was performed on an MTS Criterion tensile tester Model 43 according to ASTM D1708-13 method with a crosshead speed of 5 inch·min⁻¹ in an atmospheric condition at room temperature. Samples were cut using an ASTM die into dog-bone shapes from polymer films, which were compression molded at approximately 210 °C from precipitated and dried polymers. Tear strength tests was done according to ASTM D624-00(2012).

Attenuated total reflection (ATR) Fourier-transform infrared spectroscopy (FTIR) experiments were conducted at ambient conditions on a Bruker Tensor 27 with a Ge crystal using 32 scans at a resolution of 4 cm⁻¹.

Scanning electron microscopy (SEM) was conducted at room temperature using a Jeol IT300 scanning electron microscope under high vacuum. The samples were sputter-coated with gold using a Denton Desk II sputter coater.

### Example 1 (Comparative)

### Preparation of Polyisobutylene-Based Polyurethane

Polyurethane (PU) having PIB as soft segment with no other soft segment residues, and MDI/BDO as hard segment was synthesized using a prepolymer method. The ratio of soft segment to hard segment was varied from 70:30 to 60:40. For example, a solution containing toluene (40 mL, anhydrous) and PIB diol (Scheme 1.1, 12.1 g, 3.8 mmol) was slowly added into a preheated flask (90 °C), which contained a stir bar and MDI (6.2 g, 24.8 mmol), under N₂ atmosphere. After all PIB diols were reacted with MDI, a solution containing DMAc (40 mL, anhydrous) and BDO (1.8 g, 20.5 mmol) was then slowly added into the flask. The polymerization was then carried out at 100 °C for 20 hours. The PU products (19.8 g, yield: 98.5%) were precipitated from methanol and then dried in a vacuum oven at 50 °C. Their molecular weights and tensile properties are described in Table 1.

### Example 2 (Comparative)

### Preparation of Polyisobutylene/Polyether-Based Polyurethane

Polyurethane (PU) having mixtures of PIB and PTMO as soft segment, and MDI/BDO as hard segment, was synthesized using a prepolymer method. The ratio of soft segment to hard segment was varied from 70:30 to 65:35. For example, a solution containing toluene (40 mL, anhydrous), PIB diol (Scheme1.1, 10.2 g, 3.2 mmol), and PTMO diol (Scheme 1.2, 2.0 g, 2.0 mmol) was slowly added into a preheated flask (90 °C), which contained a stir bar and MDI (4.2 g, 16.8 mmol), under N₂ atmosphere. After all polymeric diols were reacted with MDI, a solution containing DMAc (40 mL, anhydrous) and BDO (1.0 g, 10.7 mmol) was then slowly added into the flask. The polymerization was then carried out at 100 °C for 40 hours. The PU products (16.9 g, yield: 97.1%) were precipitated from methanol and then dried in a vacuum oven at 50 °C. Their molecular weights and tensile properties are described in Table 1.

### Example 3 (Comparative)

### Preparation of Polyisobutylene/Polycarbonate-Based Polyurethane

Polyurethane (PU) having mixtures of PIB and PC in different proportions as soft segment, and MDI/BDO as hard segment, was synthesized using a prepolymer method. The ratio of soft segment to hard segment was 70:30. For example, a solution containing toluene (40 mL, anhydrous), PIB diol (Scheme1.1, 9.7 g, 3.1 mmol), and PC diol (Scheme1.3, 1.9 g, 1.0 mmol) was slowly added into a preheated flask (90 °C), which contained a stir bar and MDI (3.9 g, 15.6 mmol), under N₂ atmosphere. After all polymeric diols were reacted with MDI, a solution containing DMAc (40 mL, anhydrous) and BDO (1.0 g, 11.2 mmol) was then slowly added into the flask. The polymerization was then carried out at 100 °C for 20 hours. The PU products (16.1 g, yield: 97.2%) were precipitated from methanol and then dried in a vacuum oven at 50 °C. Their molecular weights and tensile properties are described in Table 1.

### Example 4

### Preparation of Polyisobutylene/Poly(ether-carbonate)-Based Polyurethane

Polyurethane (PU) having mixtures of PIB and PEC in different proportions as soft segment, and MDI/BDO as hard segment, was synthesized using a prepolymer method. The ratio of soft segment to hard segment was varied from 70:30 to 65:35. For example, a solution containing toluene (40 mL, anhydrous), PIB diol (Scheme1.1, 11.57 g, 3.7 mmol), and PEC diol (Scheme1.4, 2.3 g, 1.2 mmol) was slowly added into a preheated flask (90 °C), which contained a stir bar and MDI (5.9 g, 23.4 mmol), under N₂ atmosphere. After all polymeric diols were reacted with MDI, a solution containing DMAc (40 mL, anhydrous) and BDO (1.6 g, 18.1 mmol) was then slowly added into the flask. The polymerization was then carried out at 100 °C for 20 hours. The PU products (21.0 g, yield: 98.3%) were precipitated from methanol and then dried in a vacuum oven at 50 °C. Their molecular weights and tensile properties are described in Table 1.

### Example 5 (Comparative)

### Preparation of Hydrogenated Polybutadiene/Polyether-Based Polyurethane, 85A

Polyurethane (PU) having mixtures of HPB and PTMO as soft segment, and MDI/BDO as hard segment, was synthesized using a prepolymer method. The ratio of HPB to PTMO to hard segment was 40 : 10 : 50. For example, a solution containing toluene (40 mL, anhydrous), HPB diol (8.0 g, 2.7 mmol), and PTMO diol (Scheme 1.2, 2.0 g, 2.0 mmol) was slowly added into a preheated flask (90 °C), which contained a stir bar and MDI (7.6 g, 30.4 mmol), under N₂ atmosphere. A solution containing DMAc (100 mL, anhydrous), Stannous octoate (0.012 g, 0.03 mmol) and BDO (1.0 g, 10.7 mmol) was then slowly added into the flask. The polymerization was then carried out at 100 °C for 20 hours. The PU products were precipitated from water and then dried in a vacuum oven at 50 °C. Their molecular weights and tensile properties are described in Table 1.

### Example 6

### Preparation of Hydrogenated Polybutadiene/Poly(ether-carbonate)-Based Polyurethane, 85A

Polyurethane (PU) having mixtures of HPB and PEC as soft segment, and MDI/BDO as hard segment, was synthesized using a prepolymer method. The ratio of HPB to PTMO to hard segment was 40 : 10 : 50. For example, a solution containing toluene (40 mL, anhydrous), HPB diol (8.0 g, 2.7 mmol), and PEC diol (Scheme1.4, 2.0 g, 1.0 mmol) was slowly added into a preheated flask (90 °C), which contained a stir bar and MDI (7.6 g, 30.4 mmol), under N₂ atmosphere. A solution containing DMAc (100 mL, anhydrous), Stannous octoate (0.012 g, 0.03 mmol) and BDO (2.4 g, 26.4 mmol) was then slowly added into the flask. The polymerization was then carried out at 100 °C for 20 hours. The PU products were precipitated from water and then dried in a vacuum oven at 50 °C. Their molecular weights and tensile properties are described in Table 1.

### Example 7 (Comparative)

### Preparation of Hydrogenated Polybutadiene/Polyether-Based Polyurethane, 50D

Polyurethane (PU) having mixtures of HPB and PTMO as soft segment, and MDI/BDO as hard segment, was synthesized using a prepolymer method. The ratio of HPB to PTMO to hard segment was 30 : 20 : 50. For example, a solution containing toluene (50 mL, anhydrous), HPB diol (6.0 g, 2.0 mmol), and PTMO diol (Scheme 1.2, 4.0 g, 4.0 mmol) was slowly added into a preheated flask (90 °C), which contained a stir bar and MDI (7.8 g, 31.0 mmol), under N₂ atmosphere. A solution containing NMP (50 mL, anhydrous), Stannous octoate (0.013 g, 0.03 mmol) and BDO (2.2 g, 24.7 mmol) was then slowly added into the flask. The polymerization was then carried out at 100 °C for 20 hours. The PU products were precipitated from water and then dried in a vacuum oven at 50 °C. Their molecular weights and tensile properties are described in Table 1.

### Example 8

### Preparation of Hydrogenated Polybutadiene/Poly(ether-carbonate)-Based Polyurethane, 50D

Polyurethane (PU) having mixtures of HPB and PEC as soft segment, and MDI/BDO as hard segment, was synthesized using a prepolymer method. The ratio of HPB to PTMO to hard segment was 30 : 20 : 50. For example, a solution containing toluene (50 mL, anhydrous), HPB diol (6.0 g, 2.0 mmol), and PEC diol (Scheme1.4, 4.0 g, 2.0 mmol) was slowly added into a preheated flask (90 °C), which contained a stir bar and MDI (7.7 g, 30.6 mmol), under N₂ atmosphere. A solution containing NMP (50 mL, anhydrous), Stannous octoate (0.012 g, 0.03 mmol) and BDO (2.4 g, 26.3 mmol) was then slowly added into the flask. The polymerization was then carried out at 100 °C for 20 hours. The PU products were precipitated from water and then dried in a vacuum oven at 50 °C. Their molecular weights and tensile properties are described in Table 1.

**Table 1. PIB-PU's Formulations, Molecular Weights, and Mechanical Properties**

| Polymer | Mₙ | M_{w} | Ultimate Tensile Strength | Strain at Break | Tear Strength |
|---|---|---|---|---|---|
| | g·mol⁻¹ | g·mol⁻¹ | MPa | % | lbf·inch⁻¹ (Ncm⁻¹) |
| PIB-PC-PU (comparative) | 57k | 115k | 18 | 336 | 396 (639.5) |
| PIB-PTMO-PU (comparative) | 85k | 161k | 20 | 420 | 265 (464) |
| PIB-PU (comparative) | 54k | 111k | 21 | 416 | 420 (735.5) |
| PIB-PEC-PU | 71k | 131k | 20 | 385 | 505 (884.4) |
| HPB-PTMO-PU, 85A(comparative) | 110k | 140k | 34 | 335 | 461 (807) |
| HPB-PEC-PU, 85A | 80k | 95k | 37 | 289 | 516 (903.7) |
| HPB-PTMO-PU, 50D (comparative) | 151k | 260k | 29 | 275 | 782(1369.5) |
| HPB-PEC-PU, 50D | 155k | 260k | 38 | 327 | 828 (1450) |

### Example 9

### Accelerated Aging Studies

The chemical stability of the polymers of Examples 1-4 in hydrolytic, acidic, basic, and oxidative conditions was tested using an arrangement of accelerated aging conditions.

Hydrolytic Stability: To test the hydrolytic stability, compression-molded polyisobutylene polyurethane (PIB-PEC-PU, PIB-PTMO-PU, PIB-PU) was aged at 85 °C in phosphate-buffered saline (137 mM NaCl, 2.7 mM KCl, 10 mM Na₂HPO₄, 1.8 mM KH₂PO₄). After 8 weeks and 16 weeks, gel permeation chromatography was used to characterize molecular weight changes of the 85 °C polymer. The molecular weight changes are shown in Table 2.

Acidic Stability: To test the acidic stability, compression-molded polyisobutylene polyurethane (PIB-PEC-PU, PIB-PTMO-PU, PIB-PU) was treated using a 1M HCl solution at 37 °C for 8 and 16 weeks. Gel permeation chromatography was used to characterize molecular weight changes. The molecular weight changes are shown in Table 2.

Basic Stability: To test the basic stability, compression-molded polyisobutylene polyurethane (PIB-PEC-PU, PIB-PTMO-PU, PIB-PU) was treated using a 1M NaOH solution at 37 °C for 8 and 16 weeks. Gel permeation chromatography was used to characterize molecular weight changes. The molecular weight changes are shown in Table 2.

Oxidative Stability: To test the oxidative stability, compression-molded polyisobutylene polyurethane (PIB-PEC-PU, PIB-PTMO-PU, PIB-PU) was treated using a solution containing 20% w/w H₂O₂/0.1M CoCl₂ in water at 37°C at 8- and 16-week time points, the polymer was analyzed via gel permeation chromatography, Fourier-transform infrared spectroscopy, and scanning electron microscopy. The molecular weight changes are shown in Table 2.

**Table 2. Remaining Percentage of Weight Average Molecular Weight (Mw) of Polymers Upon 8- and 16-Week Exposure to the Above Solutions**

| Polymer | Aging Time (weeks) | 85°C PBS | 1M HCl | 1M NaOH | 20% w/w H₂O₂/0.1M CoCl₂ |
|---|---|---|---|---|---|
| PIB-PEC-PU | 8 | 67.9 | 97.9 | 93.7 | 78.0 |
| PIB-PTMO-PU | 8 | 67.5 | 97.3 | 102.1 | 48.4 |
| PIB-PU | 8 | 86.6 | 89.4 | 95.9 | 86.9 |
| PIB-PEC-PU | 16 | 58.9 | 106.3 | 92.1 | 85.1 |
| PIB-PTMO-PU | 16 | 59.9 | 97.4 | 102.5 | 50.4 |
| PIB-PU | 16 | 91.3 | 104.2 | 108.7 | 72.4 |

Changes in surface chemistry upon oxidation testing were analyzed via ATR-FTIR. No obvious changes through 8 weeks oxidation were observed.

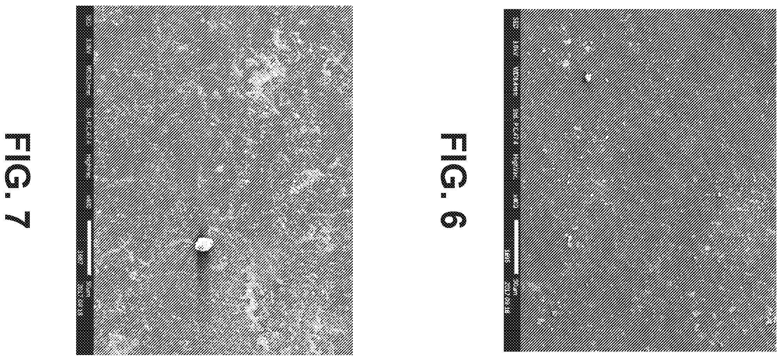

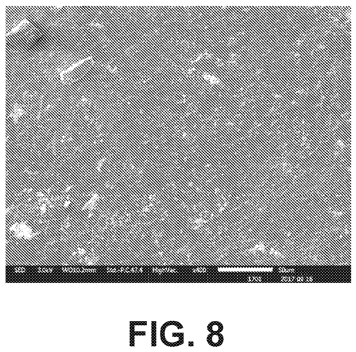

Scanning electron microscopy was used to inspect the surface for cracking potentially brought about by exposure to 20% H₂O₂/0.1M CoCl₂ solution for 8 weeks, as seen in the Figures 6-8. These images show no cracks developed on the surface of PIB-PEC PU, PIB-PTMO-PU, or PIB-PU.

It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

1. A poly(ether-carbonate)-based polymer comprising urethane, urea, or urethane-urea groups, hard segments, and soft segments, wherein the soft segments comprise poly(ether-carbonate) residues and at least one of polyisobutylene residues and hydrogenated polybutadiene residues, and the hard segments comprise diisocyanate residues and optionally chain extender residues.

2. The poly(ether-carbonate)-based polymer of claim 1 comprising urethane groups.

3. The poly(ether-carbonate)-based polymer of claim 1 or 2 wherein the hard segments comprise chain extender residues.

4. The poly(ether-carbonate)-based polymer of claim 1, 2 or 3 wherein the soft segments comprise poly(ether-carbonate) residues and polyisobutylene residues.

5. The poly(ether-carbonate)-based polymer of claim 1, 2, 3 or 4 wherein the polyisobutylene residues are derived from a polyisobutylene diol, a polyisobutylene diamine, or a combination thereof.

6. The poly(ether-carbonate)-based polymer of claim 5 wherein the polyisobutylene residues comprise aromatic-containing polyisobutylene residues.

7. The poly(ether-carbonate)-based polymer of any of the above claims wherein the weight ratio of soft segments to hard segments is within a range of 90:10 to 10:90.

8. The poly(ether-carbonate)-based polymer of any one of claims 1-6 wherein the weight ratio of polyisobutylene residues to poly(ether-carbonate) residues is within a range of 99:1 to 1:99.

9. The poly(ether-carbonate)-based polymer of any of the above claims having a weight average molecular weight of 10,000 Daltons to 2,000,000 Daltons, as determined by gel permeation chromatography with multiangle laser light scattering detection.

10. The poly(ether-carbonate)-based polymer of any of the above claims having a greater tear strength than a comparative polymer without poly(ether-carbonate) residues, wherein the comparative polymer has a shore durometer within 50% to 150% of the shore durometer of the poly(ether-carbonate)-based polymer.

11. The poly(ether-carbonate)-based polymer ofany of the above claims having greater overall chemical stability than a comparative polymer with poly(tetramethylene oxide) residues instead of poly(ether-carbonate) residues, wherein the comparative polymer has an amount of poly(tetramethylene oxide) residues equal to an amount of poly(ether-carbonate) residues in the poly(ether-carbonate)-based polymer, and wherein the comparative polymer has a shore durometer within 50% to 150% of the shore durometer of the poly(ether-carbonate)-based polymer.

12. A medical device comprising a polymeric region comprising the poly(ether-carbonate)-based polymer of any of the above claims.

13. The medical device of claim 12 comprising an implantable electrical lead, an implantable electrical pulse generator, or an implantable mechanical device.

14. The medical device of claim 13 comprising an implantable electrical lead.

15. The medical device of claim 12 wherein the polymeric region further comprises a therapeutic agent.

## Patentansprüche

1. Polymer auf Poly(ethercarbonat)-Basis, umfassend Urethan-, Harnstoff- oder Urethan-Harnstoff-Gruppen, Hartsegmente und Weichsegmente, wobei die Weichsegmente Poly(ethercarbonat)-Reste und mindestens einen von Polyisobutylen-Resten und hydrierten Polybutadien-Resten umfassen und die harten Segmente Diisocyanat-Reste und gegebenenfalls Kettenverlängerungsreste umfassen.

2. Polymer auf Poly(ethercarbonat)-Basis nach Anspruch 1, umfassend Urethangruppen.

3. Polymer auf Poly(ethercarbonat)-Basis nach Anspruch 1 oder 2, wobei die Hartsegmente Kettenverlängerungsreste umfassen.

4. Polymer auf Poly(ethercarbonat)-Basis nach Anspruch 1, 2 oder 3, wobei die Weichsegmente Poly(ethercarbonat)-Reste und Polyisobutylen-Reste umfassen.

5. Polymer auf Poly(ethercarbonat)-Basis nach Anspruch 1, 2, 3 oder 4, wobei die Polyisobutylen-Reste von einem Polyisobutylendiol, einem Polyisobutylendiamin oder einer Kombination davon stammen.

6. Polymer auf Poly(ethercarbonat)-Basis nach Anspruch 5, wobei die Polyisobutylenreste aromathaltige Polyisobutylenreste umfassen.

7. Polymer auf Poly(ethercarbonat)-Basis nach einem der vorstehenden Ansprüche, wobei das Gewichtsverhältnis von Weichsegmenten zu Hartsegmenten innerhalb eines Bereichs von 90:10 bis 10:90 liegt.

8. Polymer auf Poly(ethercarbonat)-Basis nach einem der Ansprüche 1 bis 6, wobei das Gewichtsverhältnis von Polyisobutylen-Resten zu Poly(ethercarbonat)-Resten innerhalb eines Bereichs von 99:1 bis 1:99 liegt.

9. Polymer auf Poly(ethercarbonat)-Basis nach einem der vorstehenden Ansprüche, das ein gewichtsmittleres Molekulargewicht von 10.000 Dalton bis 2.000.000 Dalton aufweist, bestimmt durch Gelpermeationschromatographie mit Mehrwinkel-Laserlichtstreuungsdetektion.

10. Polymer auf Poly(ethercarbonat)-Basis nach einem der vorstehenden Ansprüche, das eine höhere Reißfestigkeit als ein Vergleichspolymer ohne Poly(ethercarbonat)-Reste aufweist, wobei das Vergleichspolymer eine Shore-Härte innerhalb von 50 % bis 150 % der Shore-Härte des Polymers auf Poly(ethercarbonat)-Basis aufweist.

11. Polymer auf Poly(ethercarbonat)-Basis nach einem der vorstehenden Ansprüche, das eine größere chemische Gesamtstabilität als ein Vergleichspolymer mit Poly(tetramethylenoxid)-Resten anstelle von Poly(ethercarbonat)-Resten aufweist, wobei das Vergleichspolymer eine Menge an Poly(tetramethylenoxid)-Resten aufweist, die gleich einer Menge an Poly(ethercarbonat)-Resten in dem Polymer auf Poly(ethercarbonat)-Basis ist, und wobei das Vergleichspolymer eine Shore-Härte innerhalb von 50 % bis 150 % der Shore-Härte des Polymers auf Poly(ethercarbonat)-Basis aufweist.

12. Medizinische Vorrichtung, umfassend einen polymeren Bereich, der das Polymer auf Poly(ethercarbonat)-Basis nach einem der vorstehenden Ansprüche umfasst.

13. Medizinische Vorrichtung nach Anspruch 12, umfassend eine implantierbare elektrische Leitung, einen implantierbaren elektrischen Impulsgeber oder eine implantierbare mechanische Vorrichtung.

14. Medizinische Vorrichtung nach Anspruch 13, umfassend eine implantierbare elektrische Leitung.

15. Medizinische Vorrichtung nach Anspruch 12, wobei der polymere Bereich ferner ein therapeutisches Mittel umfasst.

## Revendications

1. Polymère à base de poly(éther-carbonate) comprenant des groupes uréthane, urée, ou uréthane-urée, des segments durs, et des segments mous, dans lequel les segments mous comprennent des résidus de poly(éther-carbonate) et au moins l'un parmi des résidus de polyisobutylène et des résidus de polybutadiène hydrogéné, et les segments durs comprennent des résidus de diisocyanate et facultativement des résidus d'allongeur de chaîne.

2. Polymère à base de poly(éther-carbonate) selon la revendication 1 comprenant des groupes uréthane.

3. Polymère à base de poly(éther-carbonate) selon la revendication 1 ou 2 dans lequel les segments durs comprennent des résidus d'allongeur de chaîne.

4. Polymère à base de poly(éther-carbonate) selon la revendication 1, 2 ou 3 dans lequel les segments mous comprennent des résidus de poly(éther-carbonate) et des résidus de polyisobutylène.

5. Polymère à base de poly(éther-carbonate) selon la
revendication 1, 2, 3 ou 4 dans lequel les résidus de polyisobutylène sont dérivés d'un diol de polyisobutylène, d'une diamine de polyisobutylène, ou d'une combinaison de ceux-ci.

6. Polymère à base de poly(éther-carbonate) selon la revendication 5 dans lequel les résidus de polyisobutylène comprennent des résidus de polyisobutylène contenant un aromatique.

7. Polymère à base de poly(éther-carbonate) selon l'une quelconque des revendications précédentes dans lequel le rapport pondéral des segments mous aux segments durs est dans une plage de 90:10 à 10:90.

8. Polymère à base de poly(éther-carbonate) selon l'une quelconque des revendications 1 à 6 dans lequel le rapport pondéral des résidus de polyisobutylène aux résidus de poly(éther-carbonate) est dans une plage de 99:1 à 1:99.

9. Polymère à base de poly(éther-carbonate) selon l'une quelconque des revendications précédentes ayant une masse moléculaire moyenne en poids de 10 000 Daltons à 2 000 000 Daltons, tel que déterminé par chromatographie par perméation de gel avec détection de diffusion de lumière laser multi-angle.

10. Polymère à base de poly(éther-carbonate) selon l'une quelconque des revendications précédentes ayant une plus grande résistance à la déchirure qu'un polymère comparatif sans résidus de poly(éther-carbonate), dans lequel le polymère comparatif a un duromètre Shore allant de 50 % à 150 % du duromètre Shore du polymère à base de poly(éther-carbonate).

11. Polymère à base de poly(éther-carbonate) selon l'une quelconque des revendications précédentes ayant une plus grande stabilité chimique globale qu'un polymère comparatif avec des résidus de poly(oxyde de tétraméthylène) au lieu de résidus de poly(éther-carbonate), dans lequel le polymère comparatif a une quantité de résidus de poly(oxyde de tétraméthylène) égale à une quantité de résidus de poly(éther-carbonate) dans le polymère à base de poly(éther-carbonate), et dans lequel le polymère comparatif a un duromètre Shore allant de 50 % à 150 % du duromètre Shore du polymère à base de poly(éther-carbonate).

12. Dispositif médical comprenant une région polymère comprenant le polymère à base de poly(éther-carbonate) selon l'une quelconque des revendications précédentes.

13. Dispositif médical selon la revendication 12 comprenant un fil électrique implantable, un générateur d'impulsions électriques implantable, ou un dispositif mécanique implantable.

14. Dispositif médical selon la revendication 13 comprenant un fil électrique implantable.

15. Dispositif médical selon la revendication 12 dans lequel la région polymère comprend en outre un agent thérapeutique.
